# EUROPEAN PATENT APPLICATION

(11) **EP 4 768 020 A1**
(43) Date of publication of application: **01.07.2026**
(21) Application number: 24855898.3
(22) Date of filing: 23.08.2024
(51) Int. Cl.: A61K 9/19, A61K 31/661, A61K 47/10, A61K 31/573, A61P 27/16

(54) **DEXAMETHASONE SODIUM PHOSPHATE LYOPHILIZED COMPOSITION SUITABLE FOR ADMINISTRATION TO EARDRUM AND PREPARATION METHOD THEREFOR**

(30) Priority: 24.08.2023 CN 202311076013
(71) Applicant: Heyu Suzhou Pharmaceutical Tech Co., Ltd., Suzhou, Jiangsu 215400 (CN)
(72) Inventor: WANG, Ximei, Suzhou, Jiangsu 215400 (CN); JIN, Guang, Suzhou, Jiangsu 215400 (CN); ZUO, Conglin, Suzhou, Jiangsu 215400 (CN)
(74) Representative: Schulz Junghans Patentanwälte PartGmbB
(86) International application number: PCT/CN2024/114367
(87) International publication number: WO 2025/040177

(57) **Abstract**

A dexamethasone sodium phosphate lyophilized composition suitable for administration to the eardrum and a preparation method therefor are provided. Provided is a lyophilized powder injection preparation, wherein the preparation is a dexamethasone medicament lyophilized powder injection omitting a lyophilization supporting agent or an excipient or a matrix agent, wherein the lyophilized powder injection preparation comprises pharmaceutically acceptable auxiliary material of glycerol, and the glycerol is added before the lyophilized powder injection preparation is lyophilized. The provided lyophilized preparation omits a lyophilized supporting agent or an excipient, achieving the effect of improving the stability of the drug by only adding a small amount of glycerol, and also allowing adjustment the osmotic pressure to be closer to the physiological osmotic pressure during the tympanogram administration and prolong the action time of local administration, thereby increasing the safety and effectiveness of the tympanogram administration.

## Description

### FIELD

The disclosure belongs to the field of biological medicine, and relates to a dexamethasone sodium phosphate lyophilized composition suitable for administration to the eardrum and a preparation method therefor.

### BACKGROUND

In recent years, glucocorticoids have been widely used in the treatment of various inner ear diseases such as deafness, Meniere's disease, and acute post-meningitis labyrinthitis. Sudden deafness is a sudden, non-fluctuating sensorineural deafness that occurs rapidly and may lead to sudden hearing loss in a patient within minutes, hours, or 3 days. The common accompanying symptoms are mainly tinnitus and vertigo. At present, the causes of sudden deafness are not completely clear. The main causes of sudden deafness include: microcirculation lesion of the inner ear, specific infection, immune factor, psychological factor, and the like. The acute deafness has a similar etiology to but is different from sudden deafness. The complication of the acute deafness includes tinnitus without dizziness. Some scholars believe that the etiology of the acute deafness is mainly a middle ear lesion. Both are currently treated with hormones. The hormone is helpful for interrupting the pathological injury process and protecting and promoting the recovery of various functions of the inner ears in the treatment of sudden deafness. However, it has limited therapeutic effects on the tissue and organs of the inner ears, in which irreversible damage has occurred. The hormone has an obvious curative effect on patients with sudden deafness with serious illness, and particularly has a good recovery effect on patients with intermediate frequency and low frequency. Glucocorticoids have been used internationally as the standard treatment for sudden deafness.

Dexamethasone, as an artificially synthesized adrenocortical hormone, has a strong anti-inflammatory effect on each organ system. The sodium-water retention side effect thereof is weakest, and the anti-inflammatory effect thereof is strongest, compared with hydrocortisone and derivatives thereof. Dexamethasone has a biological half-life of 36-54 h and is a long-acting glucocorticoid (*Chinese Medical Digest: Otolaryngology*, 2014, 29 (5): 280). At present, the treatment of acute deafness is mainly carried out by adopting oral dexamethasone tablets or tympanometry injection of dexamethasone sodium phosphate. However, the oral administration has obvious side effects on the whole body, and the medicine is difficult to penetrate through the blood-labyrinth barrier to reach the inner ears to take effect. In addition, the auxiliary materials in the preparation are easy to remain in the inner ears without being metabolized, thereby causing inflammation and other inner ear diseases. Many injections are sterilized by highpressure steam or circulating steam. However, the stability of the medicament is easily damaged, the interactions between raw materials and auxiliary materials are enhanced, and related substances are difficult to control. However, research proves that dexamethasone can enter the inner ears through the tympanogram pathway (Journal of Audiology and Speech Pathology, 2005, 13 (4): 260-263), so that a new idea is provided for the treatment of inner ear diseases. Compared with intravenous infusion, the total effective rate of intratympanic injection for treating sudden deafness may reach 92.5%, which is remarkably superior to intravenous administration (Jilin Medical Journal, 2013, 34 (27): 5607).

The existing commercially available dexamethasone sodium phosphate injections have low concentration and are not suitable for the requirement of a high concentration for administration to the eardrum. Furthermore, the dosing concentration problem cannot be solved by simply superimposing the lyophilizate concentration. The existing commercially available dexamethasone sodium phosphate lyophilized preparation is added with lyophilization excipients such as mannitol, lactose, sorbitol, and other auxiliary materials. In addition, in order to increase the stability of the product, substances such as antioxidants and the like are also added, and these substances are easy to remain in the ears to cause inner ear inflammation and are not suitable for tympanogram administration.

### SUMMARY

In some embodiments, the present disclosure provides a lyophilized powder injection preparation, wherein the preparation is a lyophilized powder injection of a dexamethasone drug and does not comprise a lyophilization supporting agent or an excipient or a matrix agent, the lyophilized powder injection preparation comprises a pharmaceutically acceptable auxiliary material of glycerol, and the glycerol is added before the lyophilized powder injection preparation is lyophilized.

In some embodiments, the auxiliary material further comprises a pH adjusting agent.

In some embodiments, the auxiliary material does not comprise a chelating agent or an antioxidant.

In some embodiments, the dexamethasone drug is selected from one or more of dexamethasone, a pharmaceutically acceptable derivative of dexamethasone, or a salt or an ester thereof.

In some embodiments, the dexamethasone drug comprises one or more of dexamethasone, dexamethasone sodium phosphate, dexamethasone acetate, or a salt thereof.

In some embodiments, a mass percentage of the dexamethasone sodium phosphate is about 60.0% to 99.9%.

In some embodiments, a mass percentage of the dexamethasone sodium phosphate is about 60.0% to 95%.

In some embodiments, a mass percentage of the dexamethasone sodium phosphate is about 60.0% to 90%.

In some embodiments, a mass percentage of the dexamethasone sodium phosphate is about 70.0% to 90%.

In some embodiments, the mass ratio of the dexamethasone drug to the glycerol in the lyophilized powder injection preparation is about 8:1 to 2:1.

In some embodiments, the mass ratio of the dexamethasone drug to the glycerol in the lyophilized powder injection preparation is about 8:1 to 2.5:1.

In some embodiments, the mass ratio of the dexamethasone drug to the glycerol in the lyophilized powder injection preparation is about 6:1 to 6:2.4.

In some embodiments, the mass ratio of the dexamethasone drug to the glycerol in the lyophilized powder injection preparation is about 6:1.4 to 6:2.

In some embodiments, the preparation is lyophilized from a combination solution of the dexamethasone drug, water, and a pharmaceutically acceptable auxiliary material.

In some embodiments, the dexamethasone drug is selected from one or more of dexamethasone, a pharmaceutically acceptable derivative of dexamethasone, or a salt or an ester thereof.

In some embodiments, the auxiliary material further comprises a pH adjusting agent.

In some embodiments, the osmotic pressure of the combination solution is about 60 mOsm/kg to 449 mOsm/kg.

In some embodiments, the osmotic pressure of the combination solution is about 150 mOsm/kg to 300 mOsm/kg.

In some embodiments, the osmotic pressure of the combination solution is about 240 mOsm/kg to 300 mOsm/kg.

In some embodiments, the dexamethasone drug comprises one or more of dexamethasone, dexamethasone sodium phosphate, dexamethasone acetate, or a salt thereof.

In some embodiments, the content of dexamethasone sodium phosphate in the combination solution is about 15 to 80 mg/mL.

In some embodiments, the content of dexamethasone sodium phosphate in the combination solution is about 25 to 75 mg/mL.

In some embodiments, the content of dexamethasone sodium phosphate in the combination solution is about 25 to 65 mg/mL.

In some embodiments, the content of dexamethasone sodium phosphate in the combination solution is about 20 to 55 mg/mL.

In some embodiments, the content of dexamethasone sodium phosphate in the combination solution is about 20 to 45 mg/mL.

In some embodiments, the content of dexamethasone sodium phosphate in the combination solution is about 30 mg/mL.

In some embodiments, the content of the glycerol is about 0.01% to 3% w/v.

In some embodiments, the content of the glycerol is about 0.01% to 2% w/v.

In some embodiments, the content of the glycerol is about 0.01% to 1.45% w/v.

In some embodiments, the content of the glycerol is about 0.01% to 1.2% w/v.

In some embodiments, the pH adjusting agent is selected from one or more of sodium dihydrogen phosphate, disodium hydrogen phosphate, sodium hydroxide, hydrochloric acid, citric acid, or potassium dihydrogen phosphate.

In some embodiments, the pH adjusting agent added to the combination solution is about 0.05 to 0.2 M hydrochloric acid.

In some embodiments, the lyophilized powder injection preparation is used after being redissolved by a vehicle, and the vehicle comprises water, an aqueous solution of glycerol, or an aqueous solution of sodium hyaluronate.

In some embodiments, the pH value of the combination solution is in a range of about 6 to 9.

In some embodiments, the pH value of the combination solution is in a range of about 7 to 9.

In some embodiments, the pH value of the combination solution is in a range of about 7 to 8.5.

In some embodiments, the pH value of the combination solution is in a range of about 7.5 to 8.2.

In some embodiments, the pH value of the redissolved solution is in a range of about 6 to 9.

In some embodiments, the pH value of the redissolved solution is in a range of about 7 to 9.

In some embodiments, the pH value of the redissolved solution is in a range of about 7 to 8.5.

In some embodiments, the pH value of the redissolved solution is in a range of about 7.5 to 8.2. In some embodiments, the osmotic pressure of the combination solution before lyophilization of the preparation is about 60 mOsm/kg to 449 mOsm/kg.

In some embodiments, the osmotic pressure of the combination solution before lyophilization of the preparation is about 150 mOsm/kg to 300 mOsm/kg.

In some embodiments, the osmotic pressure of the combination solution before lyophilization of the preparation is about 240 mOsm/kg to 300 mOsm/kg.

In some embodiments, the preparation is an otic injection.

In some embodiments, the preparation is an inner ear injection or a tympanic injection. There are 89 quality unqualified records in the Chinese domestic database of the sampling inspection result for the dexamethasone sodium phosphate injection sold in the market in recent years, mainly visible foreign matters and related matters. For the inner ear injection, because the body fluid and enzyme content of the inner ears are low, the metabolism is difficult, and impurities and auxiliary materials are easy to remain in the inner ear, thereby influencing the hearing. If other antioxidants or auxiliary materials are not present at all, the injection solutions are susceptible to oxidation or hydrolysis, which also affects the stability of the active ingredient.

It can be seen from the prior art that it is difficult to obtain a satisfactory dexamethasone injection with high effectiveness, good stability, and few impurities or related substances.

In the process of researching the dexamethasone injection, the inventors discovered in earlier research that in the lyophilization of dexamethasone, a lyophilized injection cannot be formed by the addition of glycerol, which is often used as a protective agent for lyophilization preservation of erythrocytes, so that the lyophilization effect of the dexamethasone is affected.

In some embodiments, it is rare that the inventors are not limited by their previous studies, and, to the finest, have obtained a preparation without a lyophilized supporting agent or an excipient, achieving the effect of improving the stability of the drug by only adding a small amount of glycerol, and also allowing adjustment the osmotic pressure to be closer to the physiological osmotic pressure during the tympanogram administration and prolong the action time of local administration, thereby increasing the safety and effectiveness of the tympanogram administration.

In some embodiments, what is especially rare is that the preparation obtained by the present disclosure is able to obtain an ideal white loose lyophilized cake without collapse of the matrix due to the addition of glycerol.

In some embodiments, the present disclosure provides a pharmaceutical kit comprising: (1) the lyophilized powder injection preparation; and (2) a vehicle; the lyophilized powder injection preparation and the solvent are separately packaged in the pharmaceutical kit.

In some embodiments, the drug delivery device of the pharmaceutical kit comprises one or more of a needle, a syringe, a pump, or a micro-injection device.

In some embodiments, the route of administration of the pharmaceutical kit comprises one or more of microsiphon/microchip administration, micropump injection administration, and reciprocating microfluidic administration.

In some embodiments, the vehicle comprises water, an aqueous solution of glycerol, or an aqueous solution of sodium hyaluronate.

In some embodiments, the present disclosure provides a method for preparing the lyophilized powder injection preparation, comprising add glycerol into water to obtain an aqueous solution of glycerol; adding dexamethasone drugs to the aqueous solution of glycerol, supplementing with water for injection, and then lyophilizing.

In some embodiments, the lyophilization process employed by the present disclosure can solve the problem of product appearance atrophy due to the addition of glycerol.

In some embodiments, the lyophilization comprises steps of: prefreezing, primary drying, and secondary drying; the temperature of the primary drying is about -50 °C to 10 °C, and the time of the primary drying is about 1000 min to 2200 min.

In some embodiments, the temperature of the primary drying is about -30 °C to 0 °C, and the time of the primary drying is about 1000 min to 2000 min.

In some embodiments, the temperature of the primary drying is about -30 °C to 0 °C, and the time of the primary drying is about 1000 min to 1800 min.

In some embodiments, the temperature of the primary drying is about -20 °C to 0 °C, and the time of the primary drying is about 1000 min to 1500 min.

In some embodiments, the primary drying comprises steps of: a temperature of -30 °C to - 15 °C and a time of about 500 min to 2000 min; increasing the temperature at a speed of 0.1 to 0.2 °C/min to -14 °C to -6 °C with a duration of 300 to 500 min; and increasing the temperature at a speed of 0.1 to 0.2 °C/min to -5 to 10 °C with a duration of 100 to 200 min.

In some embodiments, the primary drying comprises steps of: a temperature of -30 °C to - 18 °C and a time of about 500 min to 1200 min; increasing the temperature at a speed of 0.1 to 0.2 °C/min to -14 °C to -8 °C with a duration of 300 to 400 min; and increasing the temperature at a speed of 0.1 to 0.2 °C/min to -5 to 5 °C with a duration of 100 to 150 min.

In some embodiments, the temperature of the prefreezing is about -65 °C to -20 °C, and the time of prefreezing is about 50 min to 150 min.

In some embodiments, the temperature of the prefreezing is about -65 °C to -25 °C, and the time of prefreezing is about 60 min to 500 min.

In some embodiments, the temperature of the prefreezing is about -65 °C to -25 °C, and the time of prefreezing is about 670 min to 400 min.

In some embodiments, the temperature of the prefreezing is about -50 °C to -35 °C, and the time of prefreezing is about 6070 min to 200 min.

In some embodiments, the temperature of the prefreezing is about -65 °C to -25 °C, and the time of prefreezing is about 60 min to 120 min.

In some embodiments, the temperature of the prefreezing is about -50 °C to -35 °C, and the time of prefreezing is about 60 min to 120 min.

In some embodiments, the temperature of the secondary drying is about 25 °C to about 45 °C, and the time of the secondary drying is about 600 min to about 700 min.

In some embodiments, the temperature of the secondary drying is about 25 °C to about 40 °C, and the time of the secondary drying is about 600 min to about 700 min.

In some embodiments, the secondary drying comprises steps of: increasing the temperature at a speed of 1 to 6 °C/min to 25 to 50 °C with a duration of 600 to 800 min.

In some embodiments, the secondary drying comprises steps of: increasing the temperature at a speed of 1 to 3 °C/min to 25 to 40 °C with a duration of 600 to 700 min.

In some embodiments, the lyophilization further comprises a step of: applying a vacuum after the secondary drying.

In some embodiments, the vacuum is applied at 0.1 to 0.2 mbar.

In some embodiments, the time of the lyophilization is about 1500 min to 5000 min.

In some embodiments, the time of the lyophilization is about 1500 min to 4000 min.

In some embodiments, the time of the lyophilization is about 1500 min to 3000 min.

In some embodiments, the present disclosure provides a lyophilized powder injection preparation obtained by the method.

In some embodiments, the present disclosure provides the use of the lyophilized powder injection preparation, the pharmaceutical kit, or the preparation method in the manufacture of a medicament for preventing or treating an otic disease.

In some embodiments, the present disclosure provides a method for preventing or treating an ear disorder, comprising administering the lyophilized powder injection preparation or the pharmaceutical kit to a patient in need thereof.

### DETAILED DESCRIPTION

The technical solutions of the present disclosure are further illustrated by the following specific examples, which do not represent limitations to the scope of the present disclosure. Insubstantial modifications and adaptations of the disclosed concepts made by others in light of the disclosed concepts are within the scope of the disclosure.

### DEFINITIONS

As used herein, ameliorating or alleviating a symptom of a particular otic disease, disorder or condition by administration of a particular preparation or pharmaceutical kit refers to any reduction in severity, delay in onset, slowing in progression, or reduction in duration due to or caused by administration of the preparation or pharmaceutical kit, whether these effects are permanent or temporary, sustained or transient.

As used herein and in the appended set of claims, the singular forms "a", "an", and "the" include plural referents unless the context clearly dictates otherwise. Thus, for example, reference to "a method" includes a plurality of such methods, and reference to "the fragment" includes reference to one or more fragments and equivalents thereof known to those skilled in the art, and so forth.

Moreover, the use of "or" means "and/or" unless stated otherwise. Similarly, "comprising" and "including" are interchangeable and not intended to be limiting.

It should be further understood that where the term "comprising" is used to describe various embodiments, those of skill in the art will understand that in some particular instances, the language "consisting essentially of ..." or "consisting of ..." may alternatively be used to describe embodiments.

Unless otherwise defined, all terms (including technical and scientific terms) used herein have the same meaning as commonly understood by one having ordinary skill in the art to which this disclosure belongs. Although many methods and reagents are similar or equivalent to those described herein, exemplary methods and materials are disclosed herein.

It is to be understood that this disclosure is not limited to the particular methodology, protocols, reagents, etc. described herein, as such may vary. The terminology used herein is for the purpose of describing particular embodiments only and is not intended to be limiting of the disclosure.

"Antioxidants" are otically acceptable antioxidants and include, for example, butylated hydroxytoluene (BHT), sodium ascorbate, ascorbic acid, sodium metabisulfite, and tocopherol. In certain embodiments, antioxidants enhance chemical stability when desired. Antioxidants are also useful in counteracting the ototoxic effects of certain therapeutic agents, including agents used in combination with the corticosteroids disclosed herein.

"Inner ear" refers to the inner ear(s), including the cochlea and vestibular labyrinth, and the round window connecting the cochlea to the middle ear.

A "round window membrane" is a membrane that covers the cochlear window (also known as the round, right round, or round window) in humans. In humans, the thickness of the round window membrane is about 70 µm.

"Pharmacodynamics" refers to factors that determine the biological response observed at a desired site within a target otic structure relative to drug concentration.

"Pharmacokinetics" refers to the factors that determine the attainment and maintenance of an appropriate drug concentration at a desired site within the target ear structure.

The term "treating" as used herein includes prophylactically and/or therapeutically alleviating, relieving, or ameliorating the symptoms of a disease or condition, preventing other symptoms, ameliorating or preventing the underlying metabolic causes of the symptoms, inhibiting the disease or condition (e.g., arresting the development of the disease or condition), alleviating the disease or condition, causing regression of the disease or condition, alleviating the condition caused by the disease or condition, or terminating the symptoms of the disease or condition.

In some embodiments, the present disclosure provides a pharmaceutical kit for treating a tumor, comprising the lyophilized powder injection preparation and a vehicle. In a pharmaceutical kit, the lyophilized powder injection preparation are not necessarily, and usually are not, present in admixture with the vehicle, but are usually packaged separately. The lyophilized powder injection preparation and the vehicle which are separately packaged may also contain respective excipient. The excipient refers to a means which may assist the curative effect of the medicament in pharmacy. The drug kit may also comprise an independently packaged lyophilized powder injection preparation and an independently packaged vehicle.

The term "pharmaceutically acceptable" as used herein refers to those compounds, materials, compositions, and/or dosage forms which are, within the scope of sound medical judgment, suitable for use in contact with the tissues of patients without causing additional toxicity, irritation, allergic response, or other problem or complication (i.e., with a reasonable risk/benefit ratio).

The term "preventing" has art-recognized meanings that are well known in the art when used in connection with a condition, disease, syndrome, or any other medical condition and includes administering a medicament to a patient who will have a reduced frequency of occurrence of the medical condition or whose onset or symptoms will be delayed relative to a patient who will not be administered the medicament.

The term "treating" as used herein includes reversing, alleviating or inhibiting the symptoms, clinical features, and underlying pathology of a disease in a manner that ameliorates or stabilizes the condition in a patient.

The term "preventing or treating" has art-recognized meaning and includes administering one or more agents of the present disclosure to a recipient. If administered before the adverse condition is clinically manifest (e.g., a disease or other adverse condition in the recipient animal), the treatment is prophylactic (i.e., to avoid the recipient developing the adverse condition), whereas if administered after the adverse condition is clinically manifest, the treatment is therapeutic (i.e., to reduce, ameliorate, or stabilize the existing adverse condition or side effects thereof).

In the examples herein, the lyophilization technique was performed according to the protocol of the lyophilizer, and the setting of the lyophilization curve 1 (the specific details are based on curve 1 in table 13 of test example 3) was referred to in table 1 below.

**Table 1**

| **Step No.** | **Type of steps** | **Temperature (°C)** | **Vacuum (mbar)** | **Setting time (min)** | **Duration (min)** |
|---|---|---|---|---|---|
| 1 | Prefreezing | -45 | - | 1 | 120 |
| 2 | applying vacuum | - | 0.16 | - | - |
| 3 | primary drying | -20.0 | 0.16 | 60 | 900 |
| 4 | | -10.0 | 0.16 | 60 | 360 |
| 5 | | 0 | 0.16 | 60 | 120 |
| 6 | secondary drying | 30.0 | 0.16 | 60 | 660 |

### Example 1 - Formulation and preparation method of dexamethasone sodium phosphate injection

**Table 2**

| name of raw materials | amount |
|---|---|
| dexamethasone sodium phosphate | 3 g |
| glycerol | 0.4 g |
| 0.1 M HCl | q.s. |
| water for injection | q.s. to 100 mL |

Preparation method: according to the prescription table above, add glycerol into 70% of the prescribed amount of water to obtain a glycerol aqueous solution; add dexamethasone sodium phosphate to the glycerol aqueous solution, adjust the pH value of the solution to 8.0 by adding 0.1 M HCl, and add water for injection. After the medicine is completely dissolved, sterilize and filter, dispense into vials at 1 mL per vial, and half-stopper. Put the mixture into a lyophilizer for lyophilization adopting the lyophilization curve 1, and after finishing lyophilization, fill with nitrogen, full-stopper, and roll an aluminum cover to obtain the sterile lyophilized preparation.

### Example 2 - Formulation and preparation method of dexamethasone sodium phosphate injection

**Table 3**

| name of raw materials | amount |
|---|---|
| dexamethasone sodium phosphate | 3 g |
| glycerol | 0.5 g |
| 0.1 M HCl | q.s. |
| water for injection | q.s. to 100 mL |

Preparation method: according to the prescription table above, add glycerol into 70% of the prescribed amount of water to obtain a glycerol aqueous solution; add dexamethasone sodium phosphate into the glycerol aqueous solution, adjust the pH value of the solution to 8.0 by adding 0.1 M HCl, and add water for injection. After the medicine is completely dissolved, sterilize and filter, dispense into vials at 1 mL per vial, and half-stopper. Put the mixture into a lyophilizer for lyophilization adopting the lyophilization curve 1, and after finishing lyophilization, fill with nitrogen, full-stopper, and roll an aluminum cover to obtain the sterile lyophilized preparation.

### Example 3 - Formulation and preparation method of dexamethasone sodium phosphate injection

**Table 4**

| name of raw materials | amount |
|---|---|
| dexamethasone sodium phosphate | 3 g |
| glycerol | 0.7 g |
| 0.1 M HCl | q.s. |
| water for injection | q.s. to 100 mL |

Preparation method: according to the prescription table above, add glycerol into 70% of the prescribed amount of water to obtain a glycerol aqueous solution; add dexamethasone sodium phosphate into the glycerol aqueous solution, adjust the pH value of the solution to 8.0 by adding 0.1 M HCl, and add water for injection. After the medicine is completely dissolved, sterilize and filter, dispense into vials at 1 mL per vial, and half-stopper. Put the mixture into a lyophilizer for lyophilization adopting the lyophilization curve 1, and after finishing lyophilization, fill with nitrogen, full-stopper, and roll an aluminum cover to obtain the sterile lyophilized preparation.

### Example 4 - Formulation and preparation method of dexamethasone sodium phosphate injection

**Table 5**

| name of raw materials | amount |
|---|---|
| dexamethasone sodium phosphate | 3 g |
| glycerol | 1.0 g |
| 0.1 M HCl | q.s. |
| water for injection | q.s. to 100 mL |

Preparation method: according to the prescription table above, add glycerol into 70% of the prescribed amount of water to obtain a glycerol aqueous solution; add dexamethasone sodium phosphate into the glycerol aqueous solution, adjust the pH value of the solution to 8.0 by adding 0.1 M HCl, and add water for injection. After the medicine is completely dissolved, sterilize and filter, dispense into vials at 1 mL per vial, and half-stopper. Put the mixture into a lyophilizer for lyophilization adopting the lyophilization curve 1, and after finishing lyophilization, fill with nitrogen, full-stopper, and roll an aluminum cover to obtain the sterile lyophilized preparation.

### Example 5 - Formulation and preparation method of dexamethasone sodium phosphate injection

**Table 6**

| name of raw materials | amount |
|---|---|
| dexamethasone sodium phosphate | 3 g |
| glycerol | 1.2 g |
| 0.1 M HCl | q.s. |
| water for injection | q.s. to 100 mL |

Preparation method: according to the prescription table above, add glycerol into 70% of the prescribed amount of water to obtain a glycerol aqueous solution; add dexamethasone sodium phosphate into the glycerol aqueous solution, adjust the pH value of the solution to 8.0 by adding 0.1 M HCl, and add water for injection. After the medicine is completely dissolved, sterilize and filter, dispense into vials at 1 mL per vial, and half-stopper. Put the mixture into a lyophilizer for lyophilization adopting the lyophilization curve 1, and after finishing lyophilization, fill with nitrogen, full-stopper, and roll an aluminum cover to obtain the sterile lyophilized preparation.

### Example 6 - Formulation and preparation method of dexamethasone sodium phosphate injection

**Table 7**

| name of raw materials | amount |
|---|---|
| dexamethasone sodium phosphate | 3 g |
| glycerol | 1.5 g |
| 0.1 M HCl | q.s. |
| water for injection | q.s. to 100 mL |

Preparation method: according to the prescription table above, add glycerol into 70% of the prescribed amount of water to obtain a glycerol aqueous solution; add dexamethasone sodium phosphate into the glycerol aqueous solution, adjust the pH value of the solution to 8.0 by adding 0.1 M HCl, and add water for injection. After the medicine is completely dissolved, sterilize and filter, dispense into vials at 1 mL per vial, and half-stopper. Put the mixture into a lyophilizer for lyophilization adopting the lyophilization curve 1, and after finishing lyophilization, fill with nitrogen, full-stopper, and roll an aluminum cover to obtain the sterile lyophilized preparation.

### Example 7 - Formulation and preparation method of dexamethasone sodium phosphate injection (containing chelating agent calcium disodium edetate EDTA-2 NaCa)

**Table 8**

| name of raw materials | amount |
|---|---|
| dexamethasone sodium phosphate | 3.0 g |
| EDTA-2NaCa | 0.02 g |
| 0.1 M HCl | q.s. |
| water for injection | q.s. to 100 mL |

Preparation method: dissolve dexamethasone sodium phosphate and the auxiliary material EDTA-2NaCa in the prescribed amounts by water for injection under room temperature, adjust the pH value of the solution to 8.0 by adding 0.1 M HCl, and add water for injection. After the medicine is completely dissolved, sterilize and filter, dispense into vials at 1 mL per vial, and half-stopper. Put the mixture into a lyophilizer for lyophilization adopting the lyophilization curve 1, and after finishing lyophilization, fill with nitrogen, full-stopper, and roll an aluminum cover to obtain the sterile lyophilized preparation.

### Example 8 - Formulation and preparation of dexamethasone sodium phosphate for injection (containing antioxidant sodium bisulfite)

The composition of the prescription was as follows:

**Table 9**

| name of raw materials | amount |
|---|---|
| dexamethasone sodium phosphate | 3.0 g |
| sodium bisulfite | 0.02 g |
| 0.1 M NaOH | q.s. |
| water for injection | q.s. to 100 mL |

Preparation method: dissolve dexamethasone sodium phosphate and the auxiliary material sodium bisulfite in the prescribed amounts by 70% of the prescribed amount of water for injection, adjust the pH value of the solution to 8.0 by adding 0.1 M NaOH, and add water for injection. After the medicine is completely dissolved, sterilize and filter, dispense into vials at 1 mL per vial, and half-stopper. Put the mixture into a lyophilizer for lyophilization adopting the lyophilization curve 1, and after finishing lyophilization, fill with nitrogen, full-stopper, and roll an aluminum cover to obtain the sterile lyophilized preparation.

### Example 9 - Formulation and preparation of dexamethasone sodium phosphate for injection (containing hyaluronic acid)

**Table 10**

| name of raw materials | amount |
|---|---|
| dexamethasone sodium phosphate | 3.0 g |
| hyaluronic acid | 1.0 g |
| 0.1 M HCl | q.s. |
| water for injection | q.s. to 100 mL |

Preparation method: dissolve dexamethasone sodium phosphate and the auxiliary material hyaluronic acid in the prescribed amounts by 70% of water for injection, adjust the pH value of the solution to 8.0 by adding 0.1 M HCl, and add water for injection. After the medicine is completely dissolved, sterilize and filter, dispense into vials at 1 mL per vial, and half-stopper. Put the mixture into a lyophilizer for lyophilization adopting the lyophilization curve 1, and after finishing lyophilization, fill with nitrogen, full-stopper, and roll an aluminum cover to obtain the sterile lyophilized preparation.

### Test Example 1 - Inspection of appearance and osmotic pressure of lyophilized product

The finished dexamethasone sodium phosphate preparations (lyophilized powder injection preparation) prepared in Examples 1 to 9 were taken, and water content, osmotic pressure and appearance thereof were respectively inspected. Results are shown in Table 11.

**Table 11**

| Index(es) | Example 1 | Example 2 | Example 3 | Example 4 | Example 5 | Example 6 | Example 7 | Example 8 | Example 9 |
|---|---|---|---|---|---|---|---|---|---|
| appearance | white loose lyophilized cake | white loose lyophilized cake | white loose lyophilized cake | white loose lyophilized cake | White loose lyophilized cake with slightly atrophic bottom | lyophilized cake collapsed and atrophied | white loose lyophilized cake | white loose lyophilized cake | unshaped |
| Osmotic pressure (mOsm/kg) | 210 | 240 | 240 | 240 | 270 | 300 | 150 | 150 | 240 |

As can be seen from the results of appearance and osmotic pressure in the table above, in Examples 1 to 6 with glycerol added, when the content of glycerol added became larger, the effect on the formation of the preparation was larger. When the ratio of dexamethasone sodium phosphate to glycerol was at 6:3, the lyophilized cake collapsed.

Formation of the preparation of Example 9 was severely affected by the addition of hyaluronic acid. In addition, glycerol is an osmotic pressure regulator. The osmotic pressure of the preparation without addition of glycerol was obviously low and was only about 150 mOsm/kg. When the ratio of dexamethasone sodium phosphate to the glycerol reached 6:1 to 6:3, the osmotic pressure was 240 to 300 mOsm/kg and was close to the physiological osmotic pressure.

### Test Example 2 - Inspection of related substances in high-temperature test of lyophilized product

Taking the final products of dexamethasone sodium phosphate preparations prepared in the Examples 1 to 8, removing the outer packages, respectively placing in a high-temperature (60 °C) stability test box for inspection, sampling on the 0^{th} day, the 5^{th} day and the 10^{th} day respectively, and inspecting the content of related substances. (Including: degraded dexamethasone as impurity and total impurity, content). Results are shown in Table 12.

**Table 12**

| Index(es) | Time | Example 1 | Example 2 | Example 3 | Example 4 | Example 5 | Example 6 | Example 7 | Example 8 |
|---|---|---|---|---|---|---|---|---|---|
| dexamethas one | 0 d | 0.03 | 0.04 | 0.05 | 0.04 | 0.05 | 0.06 | 0.07 | 0.04 |
| | high temperature 5 d | 0.87 | 1.65 | 0.68 | 0.49 | 0.43 | 0.38 | 1.16 | 1.21 |
| | high temperature 10 d | 1.68 | 1.10 | 1.06 | 1.02 | 1.10 | 0.98 | 2.21 | 2.26 |
| total impurities | 0 d | 0.18 | 0.18 | 0.18 | 0.19 | 0.19 | 0.18 | 0.28 | 0.26 |
| | high temperature 5 d | 3.42 | 1.21 | 1.05 | 0.90 | 0.80 | 0.82 | 6.81 | 5.41 |
| | high temperature 10 d | 4.50 | 1.95 | 1.71 | 1.66 | 1.32 | 1.33 | 9.68 | 8.53 |
| dexamethas one sodium phosphate content | 0 d | 99.8% | 100.2% | 99.5% | 99.8% | 99.7% | 100.0% | 99.3% | 101.2% |
| | high temperature 5 d | 96.4% | 98.6% | 98.7% | 98.9% | 98.8% | 98.9% | 93.0% | 94.1% |
| | high temperature 10 d | 95.0% | 97.6% | 97.4% | 98.3% | 97.6% | 97.5% | 89.2% | 91.4% |

As can be seen from the results of the above table of total impurities and contents, the products prepared with addition of glycerol has a greatly improved stability compared with the products without addition of glycerol, an obviously reduced total impurity content compared with the prescriptions with addition of chelating agent EDTA and the prescriptions with addition of antioxidant, and an obviously higher dexamethasone sodium phosphate content compared with the prescription with addition of chelating agent EDTA and the prescription with addition of antioxidant.

### Test Example 3 - Comparison of the various process examples (Example 4, Example 10, Example 11, and Example 12)

The composition of the formulation used in test example 3 is shown in table 13 (formulation of example 4) below.

**Table 13**

| name of raw materials | amount |
|---|---|
| dexamethasone sodium phosphate | 3 g |
| glycerol | 1.0 g |
| 0.1 M HCl | q.s. |
| water for injection | q.s. to 100 mL |

Preparation method: add glycerol into 70% of the prescribed amount of water to obtain a glycerol aqueous solution; add dexamethasone sodium phosphate to the glycerol aqueous solution, adjust the pH value of the solution to 8.0 by adding 0.1 M HCl, and add water for injection. After the medicine is completely dissolved, sterilize and filter, dispense into vials at 1 mL per vial, and half-stopper. Put the mixture into a lyophilizer for lyophilization respectively adopting the lyophilization curves in the following table 14, and after finishing lyophilization, fill with nitrogen, full-stopper, and roll an aluminum cover to obtain the sterile lyophilized preparation.

**Table 14**

| Index | The lyophilized formulation with a mass ratio of dexamethasone sodium phosphate to glycerol being 6:2 | | | |
|---|---|---|---|---|
| | Example 4: lyophilization curve 1 | Example 10: lyophilization curve 2 | Example 11: lyophilization curve 3 | Example 12: lyophilization curve 4 |
| | Prefreezing: -45 °C, duration: 120 min; | Prefreezing: -45 °C, duration: 120 min; | Prefreezing: -45 °C, duration: 120 min; | Prefreezing: (gradient) 1 min decrease the temperature to -18 °C, duration: 10 min; 1 min decrease the temperature to -25 °C, duration: 10 min; 1 min decrease the temperature to -30 °C, duration: 10 min; and 30 min decrease the temperature to -40 °C, duration: 120 min. |
| | | Primary drying: temperature -10 °C, duration: 1320 min; and increase the temperature to 0 °C at the speed of 0.17 °C/min, duration: 120 min; | Primary drying: temperature -20 °C, duration 420 min; increase the temperature to -10 °C at the speed of 0.17 °C/min, duration: 360 min; and increase the temperature to 0 °C at a speed of 0.17 °C/min, duration: 120 min; | |
| lyophilization curve parameters | Primary drying: temperature -20 °C, duration 900 min; increase the temperature to -10 °C at the speed of 0.17 °C/min, duration: 360 min; and increase the temperature to 0 °C at a speed of 0.17 °C/min, duration: 120 min; | | | |
| | | Secondary drying: increase the temparature to 30 °C at a speed of 2 °C/min, duration: 660 min; | | |
| | | | | Vacuum: 0.16 mbar |
| | | | | Primary drying: 60 min increase the temperature to -20 °C, and maintain for 60 min; 60 min increase the temperature to -10 °C, and maintain for 360 min; and 60 min increase the temperature to 0 °C, and maintain for 300 min; |
| | | | Secondary drying: increase the temparature to 30 °C at a speed of 2 °C/min, duration: 660 min; | |
| | Secondary drying: increase the temparature to 30 °C at a speed of 2 °C/min, duration: 660 min; | Last step vacuum: 0.16 mbar | | |
| | | | Last step vacuum: 0.16 mbar | |
| | Last step vacuum: 0.16 mbar | | | Secondary drying: 30 min increase the temperature to 15 °C, and maintain for 180 min; |
| | | | | 30 min increase the temparature to 30 °C, and maintain for 60 min; and keeping the temperature at 30 °C for 180 min. (Last step vacuum: 0.05 mbar) |
| Total time of lyophilization | 2400 min | 2400 min | 1920 min | 1563 min |

The inspection results of appearance and moisture of the lyophilized products are shown in table 15.

**Table 15**

| Index(es) | Example 4 | Example 10: | Example 11: | Example 12: |
|---|---|---|---|---|
| appearance | white loose lyophilized cake | matrix collapse | matrix collapse | matrix collapse |
| Osmotic pressure (mOsm/kg) | 300 | 270 | 300 | 150 |
| water content (wt%) | 1.0% | 2.5% | 2.8% | 3.0% |

As can be seen from the above results, the primary drying temperature and drying time in the lyophilization curve parameters have a large influence on the 1.0% (w/v) glycerol prescription. For the primary drying, the temperature should be as low as possible, the drying time should be as long as possible, and the total lyophilization time is more than 2000 min.

### Test Example 4 - Pharmacodynamic test of dexamethasone sodium phosphate for injection (glycerol formulation)

Taking the final products of dexamethasone sodium phosphate preparation prepared in Examples 1 to 6 (the formulations contain glycerol), taking the final product of dexamethasone sodium phosphate preparation prepared in Example 7 (the formulation contains chelating agent calcium disodium edetate EDTA-2 NaCa), and add water for injection to the above-mentioned final products of preparation before the experiment to prepare dexamethasone sodium phosphate test solutions with the concentration of 30 mg/ml for later use. In addition, taking a purchased commercial product dexamethasone sodium phosphate (5 mg) for injection, and add water for injection before the experiment to prepare a dexamethasone sodium phosphate test solution with the concentration of 5 mg/ml for later use.

The efficacy of the dexamethasone sodium phosphate experimental group on sudden deafness was evaluated by adopting a noise-induced deafness model commonly used for new drug evaluation. In the meantime, the model group and the blank control water for injection group were set.

### 1. Guinea pig noise induced deafness model:

Normal hearing guinea pigs were divided into 9 groups. These groups were stimulated by white noise for 1 hour at 120 dB, and on the day of stimulation, after the animals in the administration groups being anesthetized by intraperitoneal injection by ketamine and xylazine according to body weight, 7 groups were respectively injected with different medicaments of Examples 1 to 7 in a single tympanic cavity at two sides of ears, and 1 group was injected with the commercially available medicament in a single tympanic cavity at two sides of ears. The model group was stimulated with white noise for 1 hour at 120 dB without treatment. At 15 days after stimulation, guinea pig hearing remained unrefreshed to normal hearing levels, exhibiting significant hearing loss, indicating successful noise modeling.

Auditory brainstem responses were used to assess hearing before and 15 days after noise, and the threshold (dB) was measured. As shown in Table 16, before the noise, the average hearing values of the animals in each groups were in the normal range of 21 to 26.1 dB. Immediately after noise, the hearing average value of the model group was 94 to 95 dB (total deafness), and after administration for 15 days, the hearing average value ranges of the administration group and the market product group were respectively 29 to 50 dB, which indicates that the dexamethasone sodium phosphate administration group has a certain hearing protection effect. The dexamethasone sodium phosphate group with the concentration of 30 mg/mL has better effect than the commercially available dexamethasone sodium phosphate group with the concentration of 5 mg/mL.

**Table 16. Table of evaluation of guinea pig noise induced deafness model test threshold (dB: mean ± standard deviation)**

| | before the noise | at the noise | 3 days administration | 5 days administration | 7 days administration | 10 days administration | 15 days administration |
|---|---|---|---|---|---|---|---|
| model group | 22.5±0.0 | 94.7±0.0 | 87.3±15.1 | 85.6±9.5 | 84.8±18.2 | 82.8±22.3 | 79.8±23.9 |
| Example 1 | 24.4±0.0 | 94.2±19.5 | 65.4±18.0 | 59.1±25.8 | 51.8±22.1 | 48.2±19.0 | 36.8±12.8 |
| Example 2 | 23.2±0.0 | 94.8±16.8 | 68.1±21.4 | 55.0±23.0 | 53.0±24.5 | 44.3±20.5 | 33.6±24.7 |
| Example 3 | 22.8±0.0 | 94.7±9.5 | 67.3±22.0 | 56.8±23.7 | 49.3±24.6 | 45.8±20.8 | 31.7±24.5 |
| Example 4 | 26.1±0.0 | 94.4±18.0 | 60.5±22.0 | 53.0±22.3 | 50.5±22.0 | 46.4±24.5 | 29.7±23.0 |
| Example 5 | 24.6±0.0 | 93.8±10.2 | 59.1±25.8 | 55.8±28.7 | 53.4±23.3 | 43.6±19.9 | 37.7±9.5 |
| Example 6 | 23.2±0.0 | 94.3±15.1 | 65.4±18.0 | 55.0±23.0 | 51.8±23.9 | 47.5±28.0 | 35.6±18.9 |
| Example 7 | 25.6±0.0 | 95.0±18.0 | 61.1±21.4 | 60.8±18.8 | 54.3±20.9 | 51.8±22.1 | 49.1±25.8 |
| commercial group | 21.8±0.0 | 94.8±24.0 | 74.2±19.5 | 67.2±15.9 | 54.5±28.0 | 47.7±23.0 | 40.8±21.4 |

### Test Example 5 - Inner ear residue test

Taking the final products dexamethasone sodium phosphate preparation prepared in Examples 1 to 9, and before the experiment, add water for injection to prepare a dexamethasone sodium phosphate test solution (the groups of Examples 1 to 9 in table 17) with the concentration of 30 mg/ml for later use. In addition, take a purchased commercial product dexamethasone sodium phosphate (5 mg) for injection, and add water for injection before the experiment to prepare a dexamethasone sodium phosphate test solution with the concentration of 5 mg/ml for later use (commercial group in Table 17). In the meantime, preparing a glycerol aqueous solution with the concentration of 2% w/v and a glycerol aqueous solution with the concentration of 25% w/v.

Preparation method of 2% w/v glycerol aqueous solution (2% w/v glycerol water group): take 2 g of glycerol and add water for injection to 100 mL to prepare a 2% w/v glycerol aqueous solution.

Preparation method of 25% w/v glycerol aqueous solution (25% w/v glycerol water group): take 25 g of glycerol and add water for injection to 100 mL to prepare a 2% w/v glycerol aqueous solution.

Inner ear residue test: taking guinea pigs, modeling according to the method in Test Example 4, anesthetizing, and performing single tympanic injections of the above dexamethasone sodium phosphate test solutions of different formulations, commercially available test solution, 2% w/v glycerol aqueous solution, and 25% w/v glycerol aqueous solution. The presence of residues and inflammation in the middle ears was observed after administration of 3d and 10d, respectively. As shown in Table 17, the formulation containing 2 w/v% or less than 2 w/v% of glycerol did not develop residue and inflammation in the inner ears. When the glycerol content reached 25%, significant residues and inflammation were shown in the inner ears of guinea pigs, and hearing was impaired. In addition, the formulations containing hyaluronic acid and the commercial products containing mannitol also produce residue in the inner ears in the early stage, and the formulation containing sodium bisulfite may induce inflammation in the inner ears of guinea pigs.

**Table 17. Middle ear residue and inflammatory status after single tympanometric injection of various dexamethasone sodium phosphate preparations**

| Test group | Prescription content (w/v) | 3 days middle ear residue | 10 days middle ear residue | inflammation | hearing recovery 10 days after administration |
|---|---|---|---|---|---|
| 2% w/v glycerol water | 2% glycerol | no residue | no residue | no inflammation | not recovered |
| 25% w/v glycerol water | 25% glycerol | with residue | no residue | inflammation | not recovered |
| Example 1 | dexamethasone sodium phosphate 3.0% | no residue | no residue | no inflammation | hearing recovered |
| | glycerol 0.4% | | | | |
| Example 2 | dexamethasone sodium phosphate 3.0% | no residue | no residue | no inflammation | hearing recovered |
| | glycerol 0.5% | | | | |
| Example 3 | dexamethasone sodium phosphate 3.0% | no residue | no residue | no inflammation | hearing recovered |
| | glycerol 0.7% | | | | |
| Example 4 | dexamethasone sodium phosphate 3.0% | no residue | no residue | no inflammation | hearing recovered |
| | glycerol 1.0% | | | | |
| Example 5 | dexamethasone sodium phosphate 3.0% | no residue | no residue | no inflammation | hearing recovered |
| | glycerol 1.2% | | | | |
| Example 6 | dexamethasone sodium phosphate 3.0% | no residue | no residue | no inflammation | hearing recovered |
| | glycerol 1.5% | | | | |
| Example 7 | dexamethasone sodium phosphate 3.0% | no residue | no residue | no inflammation | hearing recovered |
| | EDTA-2NaCa 0.02% | | | | |
| Example 8 | dexamethasone sodium phosphate 3.0% | no residue | no residue | inflammation | hearing not recovered |
| | sodium bisulfite 0.02% | | | | |
| Example 9 | dexamethasone sodium phosphate 3.0% | with residue | with residue | inflammation | hearing not recovered |
| | hyaluronic acid 1.0% | | | | |
| commercial group | dexamethasone sodium phosphate 0.5% | with residue | with residue | no inflammation | hearing recovered |
| | mannitol 20% | | | | |

## Claims

1. A lyophilized powder injection preparation, wherein the preparation is a lyophilized powder injection of a dexamethasone drug and does not comprise a lyophilization supporting agent or an excipient or a matrix agent, the lyophilized powder injection preparation comprises pharmaceutically acceptable auxiliary material of glycerol, and the glycerol is added before the lyophilized powder injection preparation is lyophilized.

2. The lyophilized powder injection preparation of claim 1, wherein the auxiliary material further comprises a pH adjusting agent;
preferably, the auxiliary material does not comprise a chelating agent or an antioxidant;
preferably, the dexamethasone drug is selected from one or more of dexamethasone, a pharmaceutically acceptable derivative of dexamethasone, or a salt or an ester thereof;
preferably, the dexamethasone drug comprises one or more of dexamethasone, dexamethasone sodium phosphate, dexamethasone acetate, or a salt thereof;
preferably, a mass percentage of the dexamethasone sodium phosphate is about 60.0% to 99.9%;
preferably, a mass percentage of the dexamethasone sodium phosphate is about 60.0% to 95%;
preferably, a mass percentage of the dexamethasone sodium phosphate is about 60.0% to 90%;
preferably, a mass percentage of the dexamethasone sodium phosphate is about 70.0% to 90%;
preferably, the mass ratio of the dexamethasone drug to the glycerol in the lyophilized powder injection preparation is about 8:1 to 2:1;
preferably, the mass ratio of the dexamethasone drug to the glycerol in the lyophilized powder injection preparation is about 8:1 to 2.5:1;
preferably, the mass ratio of the dexamethasone drug to the glycerol in the lyophilized powder injection preparation is about 6:1 to 6:2.4;
preferably, the mass ratio of the dexamethasone drug to the glycerol in the lyophilized powder injection preparation is about 6:1.4 to 6:2;

3. The lyophilized powder injection preparation of any one of claims 1 to 2, wherein the lyophilized powder injection preparation is lyophilized from a combination solution of the dexamethasone drug, water, and the pharmaceutically acceptable auxiliary material;
the dexamethasone drug is selected from one or more of dexamethasone, a pharmaceutically acceptable derivative of dexamethasone, or a salt or an ester thereof.
preferably, the auxiliary material further comprises a pH adjusting agent;
preferably, the dexamethasone drug comprises one or more of dexamethasone, dexamethasone sodium phosphate, dexamethasone acetate, or a salt thereof;
preferably, the osmotic pressure of the combination solution is about 60 to 449 mOsm/kg;
preferably, the osmotic pressure of the combination solution is about 150 to 300 mOsm/kg;
preferably, the osmotic pressure of the combination solution is about 240 mOsm/kg to 300 mOsm/kg.

4. The lyophilized powder injection preparation of any one of claims 1 to 3, wherein the content of dexamethasone sodium phosphate in the combination solution is about 15 to 80 mg/mL;
preferably, the content of dexamethasone sodium phosphate in the combination solution is about 25 to 75 mg/mL;
preferably, the content of dexamethasone sodium phosphate in the combination solution is about 25 to 65 mg/mL;
preferably, the content of dexamethasone sodium phosphate in the combination solution is about 20 to 55 mg/mL;
preferably, the content of dexamethasone sodium phosphate in the combination solution is about 20 to 45 mg/mL;
preferably, the content of dexamethasone sodium phosphate in the combination solution is about 30 mg/mL;
preferably, the content of the glycerol is about 0.01% to 3% w/v;
preferably, the content of the glycerol is about 0.01% to 2% w/v;
preferably, the content of the glycerol is about 0.01% to 1.45% w/v;
preferably, the content of the glycerol is about 0.01% to 1.2% w/v.

5. The lyophilized powder injection preparation of any one of claims 1 to 4, wherein the pH adjusting agent is selected from one or more of sodium dihydrogen phosphate, disodium hydrogen phosphate, sodium hydroxide, hydrochloric acid, citric acid, or potassium dihydrogen phosphate;
preferably, the pH adjusting agent added into the combination solution is about 0.05 to 0.2 M hydrochloric acid;
preferably, the pH value of the combination solution is in a range of about 6 to 9;
preferably, the pH value of the combination solution is in a range of about 7 to 9;
preferably, the pH value of the combination solution is in a range of about 7 to 8.5;
preferably, the pH value of the combination solution is in a range of about 7.5 to 8.2;
preferably, the lyophilized powder injection preparation is used after being redissolved by a vehicle, wherein the vehicle comprises water, an aqueous solution of glycerol, or an aqueous solution of sodium hyaluronate.

6. The lyophilized powder injection preparation of any one of claims 1 to 5, wherein a pH value of redissolved solution of the lyophilized powder injection preparation is about 6 to 9;
preferably, the pH value of the redissolved solution is in a range of about 7 to 9;
preferably, the pH value of the redissolved solution is in a range of about 7 to 8.5;
preferably, the pH value of the combination solution is in a range of about 7.5 to 8.2;
preferably, the osmotic pressure of the combination solution before the lyophilization of the lyophilized powder injection preparation is about 60 mOsm/kg to 449 mOsm/kg;
preferably, the osmotic pressure of the combination solution before the lyophilization of the lyophilized powder injection preparation is about 150 mOsm/kg to 300 mOsm/kg;
preferably, the osmotic pressure of the combination solution before the lyophilization of the lyophilized powder injection preparation is about 240 mOsm/kg to 300 mOsm/kg;
preferably, the preparation is an otic injection;
preferably, the preparation is an inner ear injection or a tympanic injection.

7. A pharmaceutical kit, comprising:
(1) the lyophilized powder injection preparation of any one of claims 1 to 6;
(2) a vehicle;
the lyophilized powder injection preparation and the vehicle in the medicine set are separately packaged;
preferably, the drug delivery device of the pharmaceutical kit comprises one or more of a needle, a syringe, a pump, or a micro-injection device;
preferably, the route of administration of the pharmaceutical kit comprises one or more of microsiphon/microchip administration, micropump injection administration and reciprocating microfluidic administration;
preferably, the vehicle comprises water, an aqueous solution of glycerol, or an aqueous solution of sodium hyaluronate.

8. A method for preparing the lyophilized powder injection preparation of any one of claims 1 to 6, wherein the method comprises steps of: add glycerol into water to obtain an aqueous solution of glycerol; adding the dexamethasone drug into the aqueous solution of glycerol, and adding water, and then lyophilizing;
the lyophilization comprises steps of: prefreezing, primary drying, and secondary drying; a temperature of the primary drying is about -50 °C to 10 °C, and a time of the primary drying is about 1000 min to 2200 min;
preferably, the temperature of the primary drying is about -30 °C to 0 °C, and the time of the primary drying is about 1000 min to 2000 min;
preferably, the temperature of the primary drying is about -30 °C to 0 °C, and the time of the primary drying is about 1000 min to 1800 min;
preferably, the temperature of the primary drying is about -20 °C to 0 °C, and the time of the primary drying is about 1000 min to 1500 min;
preferably, the primary drying comprises steps of: a temperature of -30 °C to -15 °C and a time of about 500 min to 2000 min; increasing the temperature at a speed of 0.1 to 0.2 °C/min to -14 °C to -6 °C with a duration of 300 to 500 min; and increasing the temperature at a speed of 0.1 to 0.2 °C/min to -5 to 10 °C with a duration of 100 to 200 min;
preferably, the primary drying comprises steps of: a temperature of -30 °C to -18 °C and a time of about 500 min to 1200 min; increasing the temperature at a speed of 0.1 to 0.2 °C/min to -14 °C to -8 °C with a duration of 300 to 400 min; and increasing the temperature at a speed of 0.1 to 0.2 °C/min to -5 to 5 °C with a duration of 100 to 150 min.

9. The method of claim 8, wherein a temperature of the prefreezing is about -65 °C to - 20 °C, and a time of prefreezing is about 50 min to 150 min;
preferably, the temperature of the prefreezing is about -65 °C to -25 °C, and the time of prefreezing is about 60 min to 500 min;
preferably, the temperature of the prefreezing is about -65 °C to -25 °C, and the time of prefreezing is about 70 min to 400 min;
preferably, the temperature of the prefreezing is about -50 °C to -35 °C, and the time of prefreezing is about 70 min to 200 min;
preferably, a temperature of the secondary drying is about 25 °C to about 45 °C, and a time of the secondary drying is about 600 min to about 700 min;
preferably, the temperature of the secondary drying is about 25 °C to about 40 °C, and the time of the secondary drying is about 600 min to about 700 min;
preferably, the secondary drying comprises steps of: increasing the temperature at a speed of 1 to 6 °C/min to 25 to 50 °C with a duration of 600 to 800 min;
preferably, the secondary drying comprises steps of: increasing the temperature at a speed of 1 to 3 °C/min to 25 to 40 °C with a duration of 600 to 700 min;
preferably, the lyophilization further comprises a step of: applying a vacuum after the secondary drying;
preferably, the vacuum is applied at 0.1 to 0.2 mbar;
preferably, a time of the lyophilization is about 1500 min to 5000 min;
preferably, the time of the lyophilization is about 1500 min to 4000 min;
preferably, the time of the lyophilization is about 1500 min to 3000 min.

10. Use of the lyophilized powder injection preparation according to any one of claims 1 to 6, the pharmaceutical kit according to claim 7, or the preparation method according to claim 8 or 9, in the manufacture of a medicament for the preventing or treating an otic disease.
